# EUROPEAN PATENT APPLICATION

(11) **EP 4 729 076 A1**
(43) Date of publication of application: **22.04.2026**
(21) Application number: 24825240.5
(22) Date of filing: 18.06.2024
(51) Int. Cl.: A61K 49/04, A61K 9/08, A61K 33/18, A61K 31/045, A61P 9/00, A61P 35/00

(54) **IODINE CONTRAST AGENT COMPOSITION**

(30) Priority: 19.06.2023 CN 202310728453
(71) Applicant: Chengdu Brilliant Pharmaceutical Co., Ltd., Chengdu, Sichuan 610094 (CN)
(72) Inventor: HUANG, Haoxi, Chengdu, Sichuan 610094 (CN); FAN, Xindong, Chengdu, Sichuan 610094 (CN); WANG, Deming, Chengdu, Sichuan 610094 (CN); SU, Lixin, Chengdu, Sichuan 610094 (CN); SHEN, Yuchen, Chengdu, Sichuan 610094 (CN); FENG, Sheng, Chengdu, Sichuan 610094 (CN); SUN, Chunlin, Chengdu, Sichuan 610094 (CN); SHANG, Guoning, Chengdu, Sichuan 610094 (CN); TANG, Huan, Chengdu, Sichuan 610094 (CN); YANG, Wei, Chengdu, Sichuan 610094 (CN); ZHANG, Langxi, Chengdu, Sichuan 610094 (CN); SU, Zhonghai, Chengdu, Sichuan 610094 (CN)
(74) Representative: Aera A/S
(86) International application number: PCT/CN2024/099883
(87) International publication number: WO 2024/260346

(57) **Abstract**

The present application discloses an iodine contrast agent composition comprising an iodine contrast agent, ethanol, and water, wherein based on the volume of the pharmaceutical composition, the content of the iodine contrast agent is 208 mg/mL to 624 mg/mL, and the content of the ethanol is 484 mg/mL to 677 mg/mL. This composition provides good vascular embolization and in vitro and in vivo imaging effects, which can meet the clinical requirements of image visualization. The solution is clear, homogeneous, stable, safe, and causes low irritation and has minimal impact on the tissues surrounding the lesion.

## Description

### TECHNICAL FIELD

The present invention relates to the field of pharmaceutical technology, specifically to an iodine contrast agent-ethanol composition and a preparation method and application thereof.

### BACKGROUND

Venous malformations are relatively common congenital condition of abnormal venous development, with an incidence rate of 0.01-0.02% and no significant gender difference. They may involve anywhere in the body, most commonly the oral cavity, airway, head and neck. Severe venous malformations with multi-tissue and organ involvement can lead to various disabling and fatal complications. For example, venous malformations in the head and neck may cause obvious appearance deformities, as well as functional disorders such as chewing difficulties and respiratory obstruction; venous malformations involving the limbs and joints frequetnly causes severe swelling, pain, joint stiffness, limb dysfunction, and even disability. Some patients may face life-threatening conditions due to localized intravascular coagulation (LIC) or disseminated intravascular coagulation (DIC). The mainstream international treatment for venous malformations is endovascular sclerotherapy. Its main principle is to damage and destroy vascular endothelial cells via different mechanisms, causing occlusion and fibrosis of the blood sinuses in the lesion. However, existing sclerosing agents all have significant advantages and disadvantages, making it difficult to meet the diverse and complex treatment requirements of venous malformations.

Extracranial arteriovenous malformation (AVM) is a rare, congenital high-flow vascular malformation characterized by anomalous arterial-to-venous communications. The main manifestation is that abnormally communicating arteries and veins communicate directly in the absence of a normal capillary bed, forming numerous micro-arteriovenous fistulas. AVMs commonly occur in the face, neck, and extremities, the clinical manifestations include red, swollen, hot, and pulsating masses. Most of them develop progressively, potentially leading to severe bleeding, infection, ulceration, necrosis, functional impairment, and even heart failure. Endovascular embolization plays a crucial role in the treatment of AVMs, enabling better treatment of AVMs, which was previously considered inoperable or with extremely high surgical risks.

Abdominal cystic diseases commonly involve organs such as the liver, kidneys, ovaries, and pelvic lymph nodes, with liver, kidney, and ovarian cysts being the most common ones. Their occurrence is related to a series of congenital and acquired cystic lesions. Most patients have no obvious clinical symptoms and usually require no treatment. However, as the cyst continues to grow, it may cause symptoms such as abdominal pain and back pain, and may compress and push adjacent tissue structures, thus requiring timely treatment. Previously, single-puncture drainage was used for treatment, but the recurrence rate of cysts was high. Moreover, traditional surgical operations carry significant risks, which seriously affect the prognosis of patients. With the development of imaging technologies such as computed tomography (CT) and ultrasound, image-guided percutaneous sclerotherapy has gradually replaced surgery, offering advantages such as being minimally invasive, causing less pain, repeatable, simple for operation, having good therapeutic effect, and having a lower recurrence rate than drainage sac fluid treatment. The principle of sclerotherapy is that anhydrous ethanol or other sclerosing agents come into contact with the cyst wall, leading to coagulation and denaturation of the cyst wall cell proteins, destruction of the cells, hardening and closure of the cyst wall, and cessation of cyst fluid secretion, thereby achieving the therapeutic goal of shrinking or eliminating the cyst to the maximum extent.

Intractable pain refers to persistent pain that is difficult to control with some or all treatment measures. It can be classified into cancerous pain and non-cancerous pain based on the causes. It may occur in the central and peripheral nervous systems, including any part of the body and internal organs. According to the nature of the pain, it can be divided into nociceptive pain and neuropathic pain. In some patients whose intractable pain remains unresponsive to various treatments, neurolytic therapy can be chosen under the strict control of indications and with the patient's informed consent. Nerrolysis is a medical procedure designed to manage intractable pain by disrupting the function of nerve ganglia, trunks, plexuses, or intrathecal nerves through methods such as chemical agents, radiofrequency thermocoagulation, or surgical intervention.. Ethanol (50%~100%) and phenol (5%~10%) are the most widely used agent for neurolysis under interventional guidance.

Anhydrous ethanol is a commonly used liquid embolic agent. It can rapidly destroy vascular endothelial cells and denature hemoglobin, induces extensive intravascular thrombosis and fibrosis, thereby promoting the embolization of draining veins and intralesional thrombosis formation. Its rapid dehydrating effect also leads to cellular necrosis, resulting in cyst closure and nerolysis, etc. Due to these effects, it is extensively applied in clinical practice for the interventional management of bodily cysts, arteriovenous malformations (AVMs), venous malformations, and in neurolytic therapy.

Iopromide is a non-ionic, low-osmolar contrast agent primarily used for computed tomography (CT), intra-arterial/intravenous digital subtraction angiography (DSA), and endoscopic retrograde cholangiopancreatography (ERCP)..

Anhydrous ethanol and iopromide injection may be used in combination for sclerotherapy guided by CT or digital subtraction angiography (DSA). Traditional CT or DSA-guided sclerotherapy involves two steps. First, a contrast agent (iopromide injection) is injected after puncture to fill the lesion and clarify the location and size of the lesion. Then, a non-visualizing sclerosing agent (anhydrous ethanol) is injected into the lesion cavity to replace the original contrast agent. This process is relatively cumbersome and time-consuming. Ethanol is not visible under CT or DSA and may leak from the lesion during treatment, leading to increased side effects or risks.

The existing technology involves mixing Iopromide with absolute ethanol and adding a suspending agent to form an Iopromide-absolute ethanol suspension, which streamlines the aforementioned cumbersome sclerotherapy procedure. However, as this formulation is a suspension, it carries considerable risks in clinical use..

### SUMMARY

One or more embodiments of the present application provide a pharmaceutical composition comprising an iodine contrast agent, ethanol, and water, wherein based on the volume of the pharmaceutical composition, the content of the iodine contrast agent is 208 mg/mL-624 mg/mL, and the content of the ethanol is 484 mg/mL-677 mg/mL.

In one or more embodiments, based on the volume of the pharmaceutical composition, the content of the iodine contrast agent is 220 mg/mL, 250 mg/mL, 280 mg/mL, 300 mg/mL, 350 mg/mL, 400 mg/mL, 450 mg/mL, 500 mg/mL, 550 mg/mL, or 600 mg/mL.

In one or more embodiments, based on the volume of the pharmaceutical composition, the content of the ethanol is 500 mg/mL, 550 mg/mL, 600 mg/mL, or 650 mg/mL.

In one or more embodiments, the iodine contrast agent is selected from iopromide, iobitridol, iopamidol, iomeprol, iodixanol, ioversol, iohexol, and iotrolan.

In one or more embodiments, the iodine contrast agent is iopromide or iobitridol.

In one or more embodiments, the pharmaceutical composition consists of an iodine contrast agent, ethanol, and water.

In one or more embodiments, based on the volume of the pharmaceutical composition, the content of the iodine contrast agent is 229 mg/mL-280 mg/mL.

In one or more embodiments, based on the volume of the pharmaceutical composition, the content of the ethanol is 630 mg/mL-677 mg/mL.

In one or more embodiments, based on the volume of the pharmaceutical composition, the content of the ethanol is 630 mg/mL-658mg/mL.

In one or more embodiments, the water is water for injection.

In one or more embodiments, based on the volume of the pharmaceutical composition, the content of the water is at least 29 mg/mL.

In one or more embodiments, based on the volume of the pharmaceutical composition, the content of the water is 30 mg/mL-125 mg/mL.

In one or more embodiments, based on the volume of the pharmaceutical composition, the content of the water is 40 mg/mL, 50 mg/mL, 60 mg/mL, 70 mg/mL, 80 mg/mL, 90 mg/mL, 100 mg/mL, or 120 mg/mL.

In one or more embodiments, based on the volume of the pharmaceutical composition, the content of the water is 33 mg/mL-115mg/mL.

In one or more embodiments, based on the volume of the pharmaceutical composition, the content of the water is 70 mg/mL-115mg/mL.

In one or more embodiments, based on the volume of the pharmaceutical composition, the content of the iodine is 100 mg/mL-300 mg/mL.

In one or more embodiments, based on the volume of the pharmaceutical composition, the content of the iodine is 150 mg/mL, 200 mg/mL, or 250 mg/mL.

In one or more embodiments, based on the volume of the pharmaceutical composition, the content of the iodine is 110 mg/mL-270 mg/mL.

In one or more embodiments, based on the volume of the pharmaceutical composition, the content of the iodine is 110 mg/mL-134 mg/mL.

In one or more embodiments, based on the volume of the pharmaceutical composition, the iodine contrast agent is iopromide, the content of the iopromide is 208 mg/mL-624 mg/mL (e.g., 250 mg/mL, 300 mg/mL, 350 mg/mL, 400 mg/mL, 450 mg/mL, 500 mg/mL, 550 mg/mL, or 600 mg/mL), and the content of the ethanol is 484 mg/mL-677 mg/mL (e.g., 500 mg/mL, 550 mg/mL, 600 mg/mL, or 650 mg/mL).

In one or more embodiments, based on the volume of the pharmaceutical composition, the iodine contrast agent is iopromide, the content of the iopromide is 208 mg/mL-624 mg/mL (e.g., 220 mg/mL, 250 mg/mL, 280 mg/mL, 300 mg/mL, 350 mg/mL, 400 mg/mL, 450 mg/mL, 500 mg/mL, 550 mg/mL, or 600 mg/mL), the content of the ethanol is 484 mg/mL-677 mg/mL (e.g., 500 mg/mL, 550 mg/mL, 600 mg/mL, or 650 mg/mL), and the content of the water (e.g., water for injection) is at least 29 mg/mL (e.g., 30 mg/mL-125 mg/mL, specifically, for example, 40 mg/mL, 50 mg/mL, 60 mg/mL, 70 mg/mL, 80 mg/mL, 90 mg/mL, 100 mg/mL, or 120 mg/mL). In one or more embodiments, in the pharmaceutical composition, the iodine contrast agent is completely dissolved, and the pharmaceutical composition is a homogeneous solution.

In one or more embodiments, the pharmaceutical composition is prepared as an injection.

One or more embodiments of the present application provide an injection comprising the pharmaceutical composition of the present application.

One or more embodiments of the present application provide a method for preparing the pharmaceutical composition of the present application, comprising adding an iodine contrast agent to water for injection in the contents described above, heating and stirring until completely dissolved, cooling to 50°C or a lower temperature, adding anhydrous ethanol and stirring thoroughly, and filtering to obtain the pharmaceutical composition.

One or more embodiments of the present application provide a method for preparing an injection of the present application, comprising filling and sterilizing the pharmaceutical composition of the present application to obtain the injection.

In one or more embodiments, the iodine contrast agent is selected from iopromide, iobitridol, iopamidol, iomeprol, iodixanol, ioversol, iohexol, and iotrolan.

In one or more embodiments, the iodine contrast agent is iopromide or iobitridol.

One or more embodiments of the present application provide the use of the pharmaceutical composition of the present application or the injection of the present application in the preparation of a medicament for treating vascular malformations, abdominal cystic diseases, or intractable pain.

In one or more embodiments, the vascular malformation is a venous malformation, an arteriovenous malformation, a lymphatic malformation, or a mixed malformation.

In one or more embodiments, the abdominal cystic disease is a liver cyst, kidney cyst, ovarian cyst, or pelvic lymphocytic cyst.

In one or more embodiments, the medicament for treating intractable pain is a neurolytic agent. One or more embodiments of the present application provide the pharmaceutical composition or the injection of the present application, which are used as a medicament.

One or more embodiments of the present application provide a pharmaceutical composition or the injection of the present application for use in treating vascular malformations, abdominal cystic diseases, or intractable pain.

One or more embodiments of the present application provide a method of treating vascular malformations, abdominal cystic diseases, or intractable pain, comprising administering the pharmaceutical composition or the injection of the present application to a subject in need thereof.

In one or more embodiments, the vascular malformation is a venous malformation, an arteriovenous malformation, a lymphatic malformation, or a mixed malformation.

In one or more embodiments, the abdominal cystic disease is a liver cyst, kidney cyst, ovarian cyst, or pelvic lymphocytic cyst.

In one or more embodiments, the medicament for treating intractable pain is a neurolytic agent. In one or more embodiments, in the pharmaceutical composition, the iodine contrast agent is iopromide, with a content of 229 mg/mL-280 mg/mL, the content of the ethanol is 630 mg/mL-677 mg/mL, and the content of the water for injection is 33 mg/mL-115 mg/mL

In one or more embodiments, in the pharmaceutical composition, the iodine contrast agent is iopromide, with a content of 229 mg/mL, the content of the ethanol is 630 mg/mL-658 mg/mL, and the content of the water for injection is 70 mg/mL-115 mg/mL.

This invention provides an iodine contrast agent-ethanol pharmaceutical composition, comprising an iodine contrast agent, ethanol, and water for injection, wherein the content of the ethanol is 484 mg/mL-677 mg/mL, the content of the iodine contrast agent is greater than or equal to 208 mg/mL, and the content of the water for injection is greater than 29 mg/mL;
furthermore, the composition is a homogeneous solution, wherein the iodine contrast agent is completely dissolved in the composition;
furthermore, the iodine contrast agent is selected from iopromide, iobitridol, iopamidol, iomeprol, iodixanol, ioversol, iohexol, and iotrolan, preferably iopromide or iobitridol.

This invention also provides an iodine contrast agent-ethanol pharmaceutical composition, which is composed of an iodine contrast agent, ethanol, and water for injection, wherein the content of the ethanol is 484 mg/mL-677 mg/mL, the content of the iodine contrast agent is greater than or equal to 208 mg/mL, and the content of the water for injection is greater than 29 mg/mL; the composition is a homogeneous solution, wherein the iodine contrast agent is completely dissolved in the composition; furthermore, the iodine contrast agent is selected from iopromide, iobitridol, iopamidol, iomeprol, iodixanol, ioversol, iohexol, iotrolan, and ethiodized poppyseed oil, preferably iopromide or iobitridol.

Furthermore, in the iodine contrast agent-ethanol pharmaceutical composition provided by the present invention, the content of the iodine contrast agent is preferably 208 mg/mL-624 mg/mL, more preferably 229 mg/mL-280 mg/mL.

Furthermore, in the iodine contrast agent-ethanol pharmaceutical composition provided by the present invention, the iodine content of the composition is preferably 100 mg/mL-300 mg/mL, more preferably 110 mg/mL-270 mg/mL, and the iodine content of certain specific compositions of the present invention is even more preferably 110 mg/mL-134 mg/mL.

Furthermore, in the iodine contrast agent-ethanol pharmaceutical composition provided by the present invention, the content of the ethanol is preferably 630 mg/mL-677 mg/mL, more preferably 630 mg/mL-658 mg/mL.

Furthermore, in the iodine contrast agent-ethanol pharmaceutical composition provided by the present invention, the content of the water for injection is preferably 30 mg/mL-125 mg/mL, more preferably 33 mg/mL-115 mg/mL, and in certain specific compositions of the present invention, the content of the water for injection is even more preferably 70 mg/mL-115 mg/mL. Furthermore, in the iodine contrast agent-ethanol pharmaceutical composition provided by the present invention, the iodine contrast agent is preferably iopromide with a content of 229 mg/mL-280 mg/mL, the content of the ethanol is preferably 630 mg/mL-677 mg/mL, and the content of the water for injection is preferably 33 mg/mL-115 mg/mL.

Furthermore, in the iodine contrast agent-ethanol pharmaceutical composition provided by the present invention, the iodine contrast agent is preferably iopromide with a content of 229 mg/mL, the content of the ethanol is preferably 630 mg/mL-658 mg/mL, and the content of the water for injection is preferably 70 mg/mL-115 mg/mL.

The present invention also provides a method for preparing an iodine contrast agent-ethanol pharmaceutical composition, characterized in that: comprising according to the above-mentioned prescription ratio, adding the starting material of iodine contrast agent to water for injection, heating until the iodine contrast is completely dissolved, cooling the solution to 50°C or a lower temperature, adding anhydrous ethanol and stirring evenly, filtering, filling, and sterilizing.

On the other hand, the present invention provides use of the above-described pharmaceutical composition in the treatment of vascular malformations, including venous malformations, AVMs, lymphatic malformations, and mixed malformations.

Furthermore, the use is use in the application drug in neurolytic therapy for venous malformations, arteriovenous malformation, abdominal cystic diseases, liver cysts, kidney cysts, ovarian cysts, pelvic lymphocytic cysts, and intractable pain.

In one or more embodiments, the pharmaceutical composition of the present application is a clear solution that is in solution state, has a uniform appearance, and is stable.

In one or more embodiments, the pharmaceutical composition of the present application has good solution stability; no solid precipitation is observed under conditions such as vigorous shaking or open storage.

In one or more embodiments, the pharmaceutical composition of the present application has good in vitro and in vivo imaging effects, achieving good imaging results with a low content of iodine contrast agent (e.g., iopromide).

In one or more embodiments, the pharmaceutical composition of the present application achieves a vascular embolization effect that meets clinical therapeutic requirements, realizing the purpose of visualized embolization therapy.

In one or more embodiments, the pharmaceutical composition of the present application achieves in vitro and in vivo imaging effects, allowing for clear identification of blood vessels by the naked eye, and the vascular embolization effect meets clinical treatment requirements.

In one or more embodiments, the pharmaceutical composition of the present application has minimal side effects.

In one or more embodiments, the pharmaceutical composition of the present application causes less damage to surrounding tissues than anhydrous ethanol.

In one or more embodiments, the pharmaceutical composition of the present application has good vascular embolization effects, excellent in vitro and in vivo DSA imaging effects, improves the precision of vascular embolization treatment, and achieves better therapeutic effects.

In one or more embodiments, while improving the precision of vascular embolization treatment, the pharmaceutical composition of the present application causes significantly less damage to surrounding tissues than anhydrous ethanol, and the degree of hyperplasia and/or necrosis of surrounding tissues is not significantly increased.

In one or more embodiments, the pharmaceutical composition of the present application further improves therapeutic effects, reduces the content of contrast agent, reduces usage costs, and lowers potential clinical risks, while ensuring low irritation and low adverse reactions.

In one or more embodiments, the pharmaceutical compositions of the present application (e.g., iopromide-ethanol pharmaceutical compositions, iobitridol-ethanol pharmaceutical compositions) enable dynamic monitoring during treatment, and imaging and treatment can be completed in one step with just one injection. This overcomes the cumbersome problem of traditional DSA-guided technology and sclerotherapy being performed in separate steps. Moreover, the treatment is precise, the efficacy is obvious, and the rate of adverse reaction is low.

In one or more embodiments, compared to traditional DSA-guided sclerotherapy, the pharmaceutical compositions of the present application reduce the cumbersome operation brought by the two-step injection of treatment and contrast, and only one injection is needed to complete both treatment and contrast, thus achieving the goal of dynamic monitoring during treatment.

In one or more embodiments, the pharmaceutical compositions of the present application exhibit higher treatment precision and fewer side effects, less irritation, and higher safety and stability.

In one or more embodiments, the iodine contrast agent content in the pharmaceutical compositions of the present application is significantly lower than that of the mainstream commercially available iopromide injections. It achieves good imaging effects with a lower iodine contrast agent content, thus has lower cost, and lower clinical risk.

In one or more embodiments, the pharmaceutical composition of the present application has one or more of the following good technical effects: having good vascular embolization effect; good in vivo and in vitro DSA imaging effect; being able to meet the clinical needs of image "visualization"; having a solution which is clear, homogeneous, and stable; with good safety, low irritation, and minimal impact on tissues surrounding the lesion, such as no tissue hyperplasia and/or necrosis.

### DESCRIPTION OF THE DRAWINGS

Figure 1 shows the in vitro imaging effects of Examples 5-7 and the commercially available formulation, Ultravist 300.
Figure 2 shows the in vivo imaging effects of Example 1, the commercially available formulation Ultravist 300, and normal saline.
Figure 3 shows the in vivo imaging effects of Example 1 and Comparative Example C in Experiment 3.

### DETAILED DESCRIPTION

The following examples exemplify the present application, but the scope of protection of the present application is not limited to the following examples.

### Preparation Example 1

The formulation composition of the iodine contrast agent-ethanol injection can be seen in Table 1.

**Table 1**

| **Name** | **Prescription Amount** | **Preparation Amount** |
|---|---|---|
| Iopromid | 229 mg | 22.9 kg |
| Anhydrous Ethanol | 632 mg | 63.2 kg |
| Water for Injection | 112 mg | 11.2 kg |
| Total Volume of Injection | 1 mL | 100 L |

The preparation method was as follows: The prescription amount of water for injection was added to a liquid preparation tank, the prescription amount of iopromide was slowly added, and the thus obtained mixture was stirred while heating until iopromide was completely dissolved, the solution was cooled to 50°C or a lower temperature, the prescription amount of anhydrous ethanol was added, and the resulting solution was stirred evenly. The iopromide-ethanol injection was obtained through filtration, filling and sterilization.

### Preparation Example 2

Referring to the preparation method of Example 1, the iodine contrast agent-ethanol injections of Examples 2-9 were prepared according to the formulation in Table 2 below.

**Table 2**

| **Example** | **Prescription Amount** | | | | **Preparation Amount** | | | |
|---|---|---|---|---|---|---|---|---|
| | **Iodine Contrast Agent** | **Ethanol** | **Water for Injection** | **Total Volume of injection** | **Iodine Contrast Agent** | **Ethanol** | **Water for Injection** | **Total Volume of Preparation** |
| 2 | Iopromid 229 mg | 657.3 mg | 73 mg | 1 mL | Iopromid 22.9 kg | 65.73 kg | 7.30 kg | 100 L |
| 3 | Iopromid 229 mg | 676.6 mg | 35.7 mg | 1 mL | Iopromid 22.9 kg | 67.66 kg | 3.57 kg | 100 L |
| 4 | Iopromid 623.4 mg | 484.3 mg | 123.0 mg | 1 mL | Iopromid 62.34 kg | 48.43 kg | 12.30 kg | 100 L |
| 5 | Iopromid 560.0mg | 521.4 mg | 95.3 mg | 1 mL | Iopromid 56.00 kg | 52.14 kg | 9.53 kg | 100 L |
| 6 | Iobitridol | 643.5 | 60.3 mg | 1 mL | Iobitridol | 64.35 g | 6.03 g | 100 mL |
| | 241 mg | mg | | | 24.1 g | | | |
| 7 | Iopromid 229 mg | 631 mg | 114 mg | 1 mL | Iopromid 22.9 g | 63.1 g | 11.4 g | 100 mL |
| 8 | Iopromid 236 mg | 665.7 mg | 33.7 mg | 1 mL | Iopromid 23.6 g | 66.57 g | 3.37 g | 100 mL |
| 9 | Iopromid 278 mg | 659.7 mg | 34.7 mg | 1 mL | Iopromid 27.8 g | 65.97 g | 3.47 g | 100 mL |

The iodine contrast agent-ethanol injections prepared in Examples 1-9 were all homogeneous and clear solutions. 10 mL of samples were taken from each sample, and after vigorous shaking for 30 minutes and standing, each sample solution remained homogeneous and clear. Experiments showed that pharmaceutical compositions containing iodine contrast agent, ethanol, and water for injection (e.g., ethanol content of 484 mg/mL-677 mg/mL, and iodine contrast agent content of 208 mg/mL-624 mg/mL) can yield clear solution compositions that is homogeneous and stable.

### Comparative Examples A to D

Referring to the preparation method of Example 1, the iodine contrast agent-ethanol injections were prepared according to the formulation composition in Table 3.

**Table 3**

| **Comp arative Examp le** | **Prescription Amount** | | | | **Preparation Amount** | | | |
|---|---|---|---|---|---|---|---|---|
| | **Iopro mid** | **Ethanol** | **Water for Injecti on** | **Total Volume of injection** | **Iopromi d** | **Ethan ol** | **Water for Injectio n** | **Total Volume of Preparat ion** |
| A | 229 mg | 395 mg | 399 mg | 1 mL | 22.9 g | 39.5 g | 39.9 g | 100 mL |
| B | 623.4 mg | 395 mg | 236 mg | 1 mL | 62.34 g | 39.5 g | 23.6 g | 100 mL |
| C | 200 mg | 632 mg | 126 mg | 1 mL | 20.0 g | 63.2 g | 12.2 g | 100 mL |
| D | 200 mg | 484.3 mg | 320 mg | 1 mL | 20.0 g | 48.43 g | 31.6 g | 100 mL |

Comparative Examples A to D all yielded homogeneous and clear solutions. 10 mL samples were taken from each sample, and after vigorous shaking for 30 minutes and standing, each sample solution remained homogeneous and clear.

### Comparative Example E

The iodine contrast agent-ethanol injections were prepared according to the formulation composition in Table 4.

**Table 4**

| **Name** | **Prescription Amount** | **Preparation Amount** |
|---|---|---|
| Iopromid | 229 mg | 22.90 g |
| Anhydrous Ethanol | 682.2 mg | 68.22 g |
| Water for Injection | 28.6 mg | 2.86 g |
| Total Volume of Injection | 1 mL | 100 mL |

The preparation method was the same as in Example 1. After adding 68.22 g of anhydrous ethanol to adjust to the final volume, the solution became slightly turbid. 10 mL of sample was taken, and after vigorous shaking for 30 minutes and standing, the turbidity of the sample became even more obvious. This indicates that a homogeneous and clear iodine contrast agent-ethanol solution could not be obtained from Comparative Example E.

### Comparative Example F

20 g of iopromide powder was dispersed in 50 mL (39.47 g) of anhydrous ethanol, and the mixture was shaken to obtain an iopromide-anhydrous ethanol suspension. Then, 0.05 g of Tween 80 was added to the suspension, shaken to disperse it evenly, and then anhydrous ethanol was added to adjust to the final volume to 100 mL, to obtain an iopromide-anhydrous ethanol suspension for injection.

### Comparative Example G

25 mL of commercially available iopromide injection (containing 0.769 g/mL of iopromide) was subjected to rotational evaporation to remove the solvent, and powder was obtained. Then, 25 mL (19.73 g) of anhydrous ethanol was added, and the mixture was sonicated for 20 minutes for pre-dispersion. The pre-dispersion mixture was then placed in a reactor and pressurized to 15 MPa using supercritical carbon dioxide, maintained at 40 °C and 1000 rpm/min for 2 h. Finally, the pressure was reduced to atmospheric pressure to obtain an iopromide-ethanol solution. 10 mL of sample was taken, and after vigorous shaking for 30 minutes and standing, the sample solution became slightly turbid.

### Experiment 1a

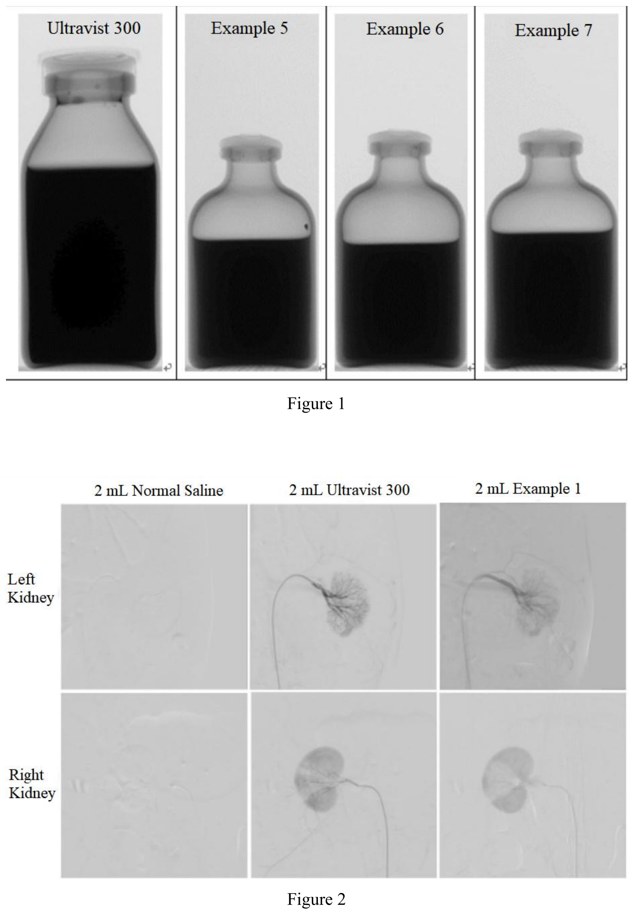

The INFX-9000C digital subtraction angiography (DSA) (Manufacturer: Canon) was used to perform in vitro imaging on Examples 5-7 and the commercially available iopromide injection (Uitravist 300). The results of imaging are shown in Figure 1.

The results of imaging show that the in vitro imaging depth of the iopromide-ethanol injection obtained in Examples 5 to 7 under DSA was not significantly different from that of the commercially available formulation (Uitravist 300). The applicant found through experiments that the pharmaceutical composition provided by the present invention, comprising iodine contrast agent, ethanol, and water for injection (e.g., ethanol content of 484 mg/mL-677 mg/mL, iodine contrast agent content of 208 mg/mL-624 mg/mL), has good in vitro imaging effect, and achieves good imaging effect with a low content of iopromide.

### Experiment 1b

2 mL each of normal saline, commercially available iopromide injection (Uitravist 300), and the iodine contrast agent-ethanol injection from Example 1 were injected into the bilateral renal arteries of New Zealand rabbits. All injections were completed within 10 seconds, and in vivo imaging was performed under DSA. The results of imaging are shown in Figure 2.

The results of imaging show that the blood vessels of the kidney were clearly visible under DSA. The iopromide-ethanol injection obtained in Example 1 enables the images of animal organs to be distinguishable to the naked eye. The applicant found through experiments that the pharmaceutical composition of the present application, comprising iodine contrast agent, ethanol, and water for injection (e.g., ethanol content of 484 mg/mL-677 mg/mL, and iodine contrast agent content of 208 mg/mL-624 mg/mL), has good in vivo imaging effects and can meet the clinical needs of image "visualization".

### Experiment 2

Eighteen New Zealand rabbits were divided into three groups, with 6 rabbits in each group. A slow intravenous injection was administered into the marginal ear vein of the right ear in each rabbit. Each group was injected with 1 mL of normal saline, 1 mL of anhydrous ethanol, and 1 mL of the iopromide-ethanol injection from Example 1, respectively. Following the injection, the injection needle was removed, and the injection site was marked. The rabbits were observed for 14 days post-administration, after which auricular tissue samples were collected for pathological staining. The degree of vascular embolism was graded and evaluated: no embolism, grade "0"; partial embolism, grade "I"; complete embolism, grade "II". Simultaneously, the degree of peripheral tisue damage was graded and evaluated: no patholigical changes were observed in the perivascular tissue, grade "0"; absent or mild increase in perivascular inflammatory cells, mild granulation tissue hyperplasia, no significant tissue thickening: grade "I"; mild increase in perivascular inflammatory cells, significant granulation tissue hyperplasia, observable tissue thickening: grade "II"; partial necrosis of perivascular tissue, necrotic area less than 1/3 of the collected tissue section: grade "III"; extensive necrosis of perivascular tissue, necrotic area accounting for 1/3 or more of the collected tissue: grade "IV".

The results of the vascular embolism and peripheral tissue damage evaluations for the three experimental groups are shown in Table 5.

**Table 5**

| **Group** | **Incidence of Venous Embolism Grade (%)** | | | **Statistical Analysis** | **Percentage of Peripheral Tissue Damage by Grade (%)** | | | | | **Statistical Analysis Results** |
|---|---|---|---|---|---|---|---|---|---|---|
| | **Grade 0** | **Grade I** | **Grade II** | | **Grade 0** | **Grade I** | **Grade II** | **Grade III** | **Grade IV** | |
| Normal Saline Group (N=6) | 100.0 | 0.0 | 0.0 | / | 100.0 | 0.0 | 0.0 | 0.0 | 0.0 | / |
| Anhydrous Ethanol Group (N=6) | 0.0 | 66.7 | 33.3 | vs.Normal Saline: p = 0.0019 | 0.0 | 0.0 | 66.7 | 0.0 | 33.3 | vs. Normal Saline: p = 0.0025 |
| Example 1 group (N=6) | 0.0 | 50.0 | 50.0 | vs. Anhydrous Ethanol: p = 0.2540 | 50.0 | 16.7 | 33.3 | 0.0 | 0.0 | vs. Anhydrous Ethanol: p = 0.0123 |

In the table, N represents the number of animals in each group. The chi-square test was used to analyze differences between groups. "/" indicates that the test is not applicable or no statistically significant differences were observed between that group and the others. p<0.05 indicates a significant difference.

Based on the above exemplary experimental results, it can be seen that the iopromide-ethanol injection of Example 1 showed no significant difference in the vascular embolization effect compared to anhydrous ethanol, but is slightly better than anhydrous ethanol in terms of the incidence of complete embolization. This indicates that the iopromide-ethanol injection of the present application provides a good vascular embolization effect, meeting the clinical requirements for treating venous malformations. Example 1 causes significantly less damage to the peripheral tissues compared to anhydrous ethanol, exhibiting lower side effects and higher treatment precision compared to anhydrous ethanol.

### Experiment 3

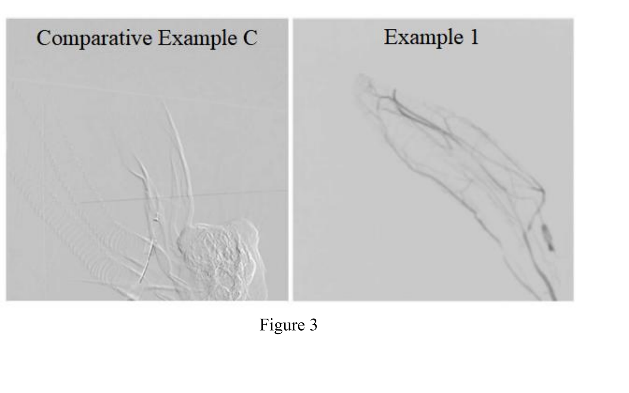

Forty New Zealand rabbits were divided into 10 groups, with 4 rabbits in each group. A slow intravenous injection was administered into the marginal ear vein of the right ear in each rabbit Each group of animals was injected with 1 mL of ioprofame-ethanol injection obtained from Examples 1, 3, 4, 6, and Comparative Examples A, B, C, D, F, respectively. Following the injection, the injection needle was removed, and the injection site was marked, and the animals in each group were subjected to in vivo imaging under DSA, and the DSA imaging intensity values were recorded. The rabbits were then observed for 14 days post-administration, after which auricular tissue samples were collected for pathological staining.. Each animal was individually scored for the degree of vascular embolism as follows: no embolism, score 0; partial embolism, score 1; complete embolism, score 2. Concurrently, each animal was individually scored for the degree of peripheral tissue damage as follows: no pathological changes were observed in perivascular tissue, score 0; absent or mild increase in perivascular inflammatory cells, mild granulation tissue hyperplasia, no significant tissue thickening, score 1; mild increase in perivascular inflammatory cells, significant granulation tissue hyperplasia, observable tissue thickening, score 2; partial necrosis of perivascular tissue, necrotic area less than 1/3 of the collected tissue section, score 3; extensive necrosis of perivascular tissue, necrotic area accounting for 1/3 or more of the collected tissue, score 4.. The average scores for vascular embolism degree and peripheral tissue damage for each group of animals were used as the venous occlusion pathological score and the peripheral tissue damage pathological score, respectively. Figure 3 shows the imaging results of the iopromide-ethanol injections of Example 1 and Comparative Example C. The experimental results for all groups are shown in Table 6.

**Table 6**

| **Group** | **Mean DSA Imaging Intensity** | **Venous Occlusion Pathological Score** | **Peripheral Tissue Pathological Score** |
|---|---|---|---|
| Example 1 | 65127 | 1.50 | 0.75 |
| Example 2 | 66287 | 1.50 | 0.75 |
| Example 3 | 67300 | 1.75 | 1.25 |
| Example 4 | 81744 | 1.00 | 0.50 |
| Example 6 | 56866 | / | / |
| Comparative Example A | 61352 | 0.25 | 0.50 |
| Comparative Example B | 82354 | 0.25 | 0.75 |
| Comparative Example C | 35814 | 1.25 | 2.00 |
| Comparative Example D | 32919 | 0.75 | 1.50 |
| Comparative Example F | 31966 | 1.75 | 2.75 |

| | | | |
|---|---|---|---|
| "/" indicates not detected. | | | |

Based on the results from the exemplary experiments above, the Venous Occlusion Pathological Scores of Comparative Examples A and B were less than 0.5, indicating a poor venous occlusion effect. The Peripheral Tissue Damage Pathological Scores of Comparative Examples C, D, and F were greater than or equal to 1.5, indicating noticeable peripheral tissue necrosis and significant side effects. Furthermore, the DSA imaging intensities of Comparative Examples C, D, and F were weak, making it difficult to visually distinguish the blood vessels.

These experiments demonstrate that the tested iopromide-ethanol injections (e.g., from Examples) exhibited good in vivo and in vitro DSA imaging effect, allowing for clear visual distinction of in vivo blood vessels. They also showed a stronger vascular embolization effect, meeting the requirements for clinical precision therapy. Furthermore, their side effects were smaller, and the damage to peripheral tissues was significantly less than that caused by anhydrous ethanol, with no significant increase in the degree of hyperplasia and necrosis.

### Experiment 4

The samples prepared in Examples 1, 3, 4, and Comparative Example G were placed under open-air and room temperature conditions to investigate the stability of each sample solution. The results are shown in Table 7.

**Table 7**

| **Sample** | **After 6 hours** | **After for 12 hous** | **After 1 Day** |
|---|---|---|---|
| Example 1 | Clear, no solid precipitation | Clear, no solid precipitation | Clear, no solid precipitation |
| Example 3 | Clear, no solid precipitation | Clear, no solid precipitation | Clear, no solid precipitation |
| Example 4 | Clear, no solid precipitation | Clear, no solid precipitation | Clear, no solid precipitation |
| Comparative Example G | Clear, no solid precipitation | Slightly turbid, slight solid precipitation | Turbid, sifnificant solid precipitation |

Based on the exemplary experiment above, Comparative Example G exhibited significant stability issues when placed under open-air conditions. The iopromide-ethanol injection of the present application demonstrates superior placement stability compared to Comparative Example G. These experiments indicate that the pharmaceutical composition of the present application, comprising an iodine-based contrast agent, ethanol, and water for injection (for instance, with an ethanol content of 484 mg/mL-677 mg/mL and an iodine contrast agent content of 208 mg/mL-624 mg/mL), remains resistant to solid precipitation even when exposed to open air, demonstrating good solution stability.

## Claims

1. A pharmaceutical composition comprising an iodine contrast agent, ethanol, and water, wherein based on the volume of the pharmaceutical composition, the content of the iodine contrast agent is 208 mg/mL-624 mg/mL, and the content of the ethanol is 484 mg/mL-677 mg/mL.

2. The pharmaceutical composition according to claim 1, wherein the iodine contrast agent is selected from iopromide, iobitridol, iopamidol, iomeprol, iodixanol, ioversol, iohexol, and iotrolan; preferably iopromide or iobitridol.

3. The pharmaceutical composition according to claim 1 or 2, wherein the pharmaceutical composition is composed of an iodine contrast agent, ethanol, and water.

4. The pharmaceutical composition according to any one of the preceding claims, wherein:
based on the volume of the pharmaceutical composition, the content of the iodine contrast agent is 229 mg/mL-280 mg/mL;
preferably, based on the volume of the pharmaceutical composition, the content of the ethanol is 630 mg/mL-677 mg/mL, preferably 630 mg/mL-658 mg/mL;
preferably, wherein the water is water for injection; more preferably, based on the volume of the pharmaceutical composition, the content of the water is at least 29 mg/mL; preferably, the content of the water is 30 mg/mL-125 mg/mL, preferably 33 mg/mL-115 mg/mL, more preferably 70 mg/mL-115 mg/mL;
preferably, based on the volume of the pharmaceutical composition, the content of the iodine is 100 mg/mL-300 mg/mL, more preferably 110 mg/mL-270 mg/mL, more preferably 110 mg/mL-134 mg/mL;
preferably, based on the volume of the pharmaceutical composition, the iodine contrast agent is iopromide, the content of the iopromide is 208 mg/mL-624 mg/mL, and the content of the ethanol is 484 mg/mL-677 mg/mL.

5. The pharmaceutical composition according to any one of the preceding claims, wherein in the pharmaceutical composition, the iodine contrast agent is completely dissolved, and the pharmaceutical composition is a homogeneous solution.

6. The pharmaceutical composition according to any one of the preceding claims, wherein the pharmaceutical composition is prepared as an injection.

7. An injection comprising the pharmaceutical composition according to any one of claims 1 to 6.

8. A method for preparing the pharmaceutical composition of any one of claims 1 to 6 or the injection of claim 7, comprising according to the contents of any one of claims 1 to 6, adding an iodine contrast agent to water for injection, heating and stirring until completely dissolved, cooling to 50°C or a lower temperature, adding anhydrous ethanol and stirring thoroughly, and filtering to obtain the pharmaceutical composition; optionally, filling and sterilizing the pharmaceutical composition to obtain the injection;
preferably, the iodine contrast agent is selected from iopromide, iobitridol, iopamidol, iomeprol, iodixanol, ioversol, iohexol, and iotrolan; preferably iopromide or iobitridol.

9. Use of the pharmaceutical composition of any one of claims 1 to 6 or the injection of claim 7 in the preparation of a medicament for treating vascular malformations, abdominal cystic diseases, or intractable pain; preferably, the vascular malformation is a venous malformation, an arteriovenous malformation, a lymphatic malformation, or a mixed malformation.

10. The use according to claim 9, wherein the abdominal cystic disease is a liver cyst, kidney cyst, ovarian cyst, or pelvic lymphocytic cyst; preferably, the medicament for treating intractable pain is a neurolytic agent.
